# EUROPEAN PATENT SPECIFICATION

(11) **EP 1 669 353 B1**
(45) Date of publication and mention of the grant of the patent: **20.05.2009**
(21) Application number: 05257405.0
(22) Date of filing: 01.12.2005
(51) Int. Cl.: C07D 317/04

(54) **Process for preparing alpha, beta - unsaturated esters**
Verfahren zur Herstellung von alpha, beta - unungesättigten Estern
Procédé de préparation d'esters alpha, beta - insaturés

(30) Priority: 03.12.2004 JP 2004350777
(43) Date of publication of application: 14.06.2006
(73) Proprietor: DAISO CO., LTD., Osaka-shi Osaka-fu (JP)
(72) Inventor: Igi, Kimitaka, c/o Daiso Co., Ltd, Osaka-shi Osaka-fu (JP); Hirata, Makoto, c/o Daiso Co., Ltd, Osaka-shi Osaka-fu (JP); Mikami, Masafumi, c/o Daiso Co., Ltd, Osaka-shi Osaka-fu (JP); Nagano, Yoshifumi, c/o Daiso Co., Ltd, Osaka-shi Osaka-fu (JP)
(74) Representative: Stoner, Gerard Patrick

(56) References cited:
- WO-A-20/05040149
- US-A- 5 821 374
- JANSON, MARIANNE; KVARNSTRÖM, INGEMAR; SVENSSON, STEFAN C. T.; CLASSON, BJÖRN; SAMUELSSON, BERTIL:: "New Routes to Isomerically Pure Cyclopentanes. Synthesis of (3S,4S)-3,4-Bis(benzoyloxymethyl)cyclopent an-1-ol using Palladium-Catalyzed [2 + 3] Cycloaddition" SYNTHESIS, vol. 1993, no. 1, 1993, pages 129-133, XP002303374 ISSN: 0040-4039
- NOOY DE A E J ET AL: "ON THE USE OF STABLE ORGANIC NITROXYL RADICALS FOR THE OXIDATION OFPRIMARY AND SECONDARY ALCOHOLS" SYNTHESIS, GEORG THIEME VERLAG, STUTTGART, DE, October 1996 (1996-10), pages 1153-1174, XP002072173 ISSN: 0039-7881
- MARTIN G. BANWELL ET ALL.: "A chemoenzymatic synthesis of the styrllactone (+)-goniodiol from naphthalene" J.CHEM.SOC., PERKIN TRANS., vol. 1, 2002, pages 1622-1624, XP002374247
- FRITZ-LANGHALS, ELKE: "Technical Production of Aldehydes by Continuous Bleach Oxidation of Alcohols Catalyzed by 4-Hydroxy- TEMPO" ORGANIC PROCESS RESEARCH & DEVELOPMENT , 9(5), 577-582 CODEN: OPRDFK; ISSN: 1083-6160, 2005, XP002374248

## Description

### TECHNICAL FIELD

The present invention relates to a process for preparing an α,β-unsaturated ester useful as an intermediate for preparing an optically active medicine.

### BACKGROUND

From of old, a glyceraldehyde acetonide, a kind of glyceraldehyde derivatives has been generally derived from natural sources and its R-isomer is derived from D-mannitol, and its S-isomer is derived from L-ascorbic acid (See Organic Synthesis, CV, 9. p.450 and Organic Synthesis, CV, 9. p.454). Furthermore, it is also known to oxidize a glycerol acetonide with an oxidizing agent such as PCC, etc. to prepare a glyceraldehyde acetonide (See Synlett, 2001, 10, p.1565).

It is reported that an α,β-unsaturated ester is derived from D-mannitol (See Japanese patent publication A 62-246567), and after converting to an aldehyde by Swern-oxidation of a glycerol acetonide, it is reacted with (triphenylphosphoranylidene)acetate to prepare an α,β-unsaturated ester (See Synthesis, 1993, p.129).

US 5,821,374 describes the oxidation of a specific group of primary and secondary alcohols to form the corresponding aldehyde or ketone using an N-chloro compound as an oxidising agent in the presence of a catalyst comprising a nitroxyl radical or nitroxyl radical derivative.

A.E.J. de Nooy et al. (Synthesis, October 1996, 1153) summarise various different oxidation reactions utilising stable organic nitroxyl radicals to oxidise primary and secondary alcohols. Different reaction schemes and conditions are discussed as well as the mechanistic aspects of the reactions.

In preparing glyceraldehyde acetonide from natural sources, it is necessary to change the method thereof according to the optical configuration (R-isomer or S-isomer) of an objective compound, and in either method expensive sodium periodate must be used. In the method for oxidizing glycerol acetonide with PCC, a toxic chromium metal must be used in more than molar equivalent. Therefore these methods are not suitable for the industrial application.

In regard to synthesis of an α,β-unsaturated ester starting from D-mannitol, only an optical-isomer of one side can be synthesized and expensive sodium periodate must be used. On the other hand, the process for preparing the glycerol acetonide by Swern-oxidation must be carried out at such a lower temperature as -60°C, and is accompanied with bad smell due to dimethylsulfide.

The present inventors have studied extensively to address the above problems and found that by oxidizing using a nitroxyl radical compound as catalyst and a co-oxidant without using a toxic oxidizing agent, glycerol acetonide or its homologous compound can be prepared under mild conditions, and that R-isomer or S-isomer of glyceraldehyde acetonide or its homologous compound is preparable by the same method, and then it is further reacted with an acetic acid ester derivative to prepare an α,β-unsaturated ester.

Thus, the present invention relates to a process for preparing an α,β-unsaturated ester of the following compound (4), 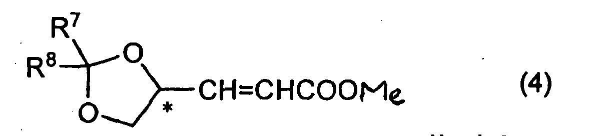 wherein R⁷ and R⁸ are methyl groups which comprises oxidizing a glycerol derivative of the following formula (2), 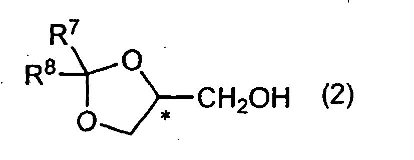 wherein R⁷ and R⁸ are the same as defined above, in the presence of a nitroxyl radical compound represented by the following (1), 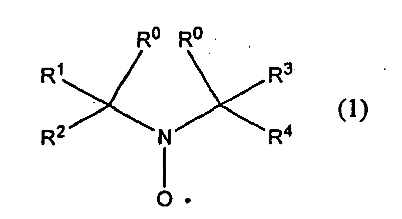 wherein R⁰ to R⁴ are the same or different and straight or branched C₁₋₁₀ alkyl group, or two R⁰s may be combined together with the nitrogen atom in the intramolecule to form a 5 to 7 membered hetero ring,
and a co-oxidant to prepare a glyceraldehyde of the following formula (3), 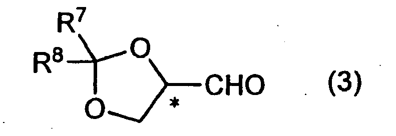 wherein R⁷ and R⁸ are the same as defined above, and then reacting the compound (3) with trimethyl phosphonoacetate to give the compound (4) without isolation of the compound (3).

By using the present process, a glyceraldehyde derivative (3) can be prepared by oxidizing a glycerol derivative (2) under relatively mild conditions and without any toxic oxidizing agent. Furthermore, an α,β-unsaturated ester (4) can be obtainable in one pot-method without isolation of a glyceraldehyde derivative (3), which is said to be generally very difficult to isolate itself after reaction.

Although the catalyst used in the present invention is a catalyst represented by the above formula (1), it may include such a compound wherein at least one of R⁰ to R⁴ represents C₁₋₆ alkyl group substituted by C₁₋₆ alkoxy group in the formula (1), as long as it is effective in the process.

Catalysts of formula (1) are known and may be prepared e.g. by the methods described in European Patents 574666 and 574667.

As the catalyst (1) a compound of the following formula (1a) is preferable, 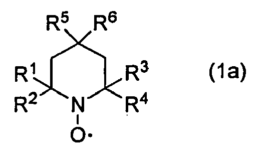 wherein R¹ to R⁴ are the same as defined above, R⁵ and R⁶ are both hydrogen atom or both C₁₋₆ alkoxy group, or one of R⁵ and R⁶ is hydrogen atom and the other is hydroxy group, C₁₋₆ alkoxy group, acyloxy group, or acylamino group,

The compound (1a) wherein R¹ to R⁴ are all methyl group, and R⁵ and R⁶ are both hydrogen atom, or one of R⁵ and R⁶ is hydrogen atom and the other is hydroxy group, methoxy group, acetoxy group, benzoyloxy group or acetamide group is especially preferable.

The amount of the catalyst (1) used in oxidation of a glycerol derivative (2) is 0.05 to 20 mole% against the glycerol derivative (2), more preferably 0.1 to 3 mole%.

Co-oxidants usable with the catalyst (1) or (1a) in oxidation of glycerol derivative (2) include a N-chloroamide compound such as N-chlorosuccinimide (NCS), dichlorodimethylhydantoin, trichloroisocyanuric acid, N-chloro-4-toluenesulfonamide or N-chloro-4-benzenesulfonamide, a N-bromoamide compound such as N-bromoacetamide or N-bromosuccinimide (NBS), chlorine, bromine, iodine, sodium hypochlorite, t-butyl hypochlorite, bleaching powder, diacetoxyiodobenzene or m-chloroperbenzoic acid (m-CPBA). Preferable ones are sodium hypochlorite, NCS, trichloroisocyanuric acid, NBS and diacetoxyiodobenzene. NCS is preferably used together with a compound having bromide ion such as sodium bromide or potassium bromide. Especially preferable ones are a N-bromoamide compound and a combination of N-chlorosuccinimide and a compound having bromide ion.

The amount of a co-oxidant is preferably 1.05 to 2.0 molar equivalents against the glycerol derivative (2).

The solvent useful in the oxidation of glycerol derivative includes a halogeno compound-solvent such as dichloromethane, chloroform or 1,2-dichloroethane, a ketone-solvent such as acetone or methylethylketone, an ester-solvent such as ethyl acetate or butyl acetate, an ether-solvent such as tetrahydrofuran (THF), 1,4-dioxane or methyl t-butyl ether (MTBE), an aromatic hydrocarbon-solvent such as benzene or toluene. The ester-solvent, the ether-solvent and the halogeno compound-solvent are preferably used.

When an acid occurs during the oxidation reaction of the present invention, a base may be used such as preferably sodium hydrogencarbonate, sodium carbonate, potassium hydrogencarbonate, potassium carbonate, sodium acetate, sodium phosphate or potassium hydrogenphosphate.

The reaction temperature depends on the kinds of co-oxidants, but preferably, -15 to 100°C, more preferably 0 to 30°C.

Thus prepared glyceraldehyde derivative (3) is not isolated and is further reacted with trimethyl phosphonoacetate to prepare the α,β-unsaturated ester (4).

In the reaction of the glyceraldehyde derivative (3) with trimethyl phosphonoacetate (Horner-Wadsworth-Emmons reaction (HWE reaction)), the same bases as generally used in HWE reaction are useful. For example, potassium carbonate, sodium carbonate, potassium hydrogencarbonate, sodium hydride, potassium hydride, potassium t-butoxide, butyllithium, lithium diisopropylamide (LDA), triethylamine, diisopropylethylamine, 1,8-diazabicyclo[5,4,0]undec-7-ene (DBU) and 1,5-diazabicyclo[4,3,0]non-5-ene (DBN) are preferably illustrated.

The amount of the base used in HWE reaction is 0.5 to 3 molar equivalents against the aldehyde derivative (3).

The reaction temperature at HWE reaction depends on the kind of base or phosphor reagents but is generally in the range of -78 to 150°C.

When R-isomer of a glycerol derivative (2) is used as a starting material, S-isomer of a glyceraldehyde derivative (3) is obtained and further R-isomer of an α,β-unsaturated ester (4) is obtained without any remarkable racemization, respectively. On the other hand, when S-isomer of a glycerol derivative (2) is used as a starting material, R-isomer of a glyceraldehyde derivative (3) is obtained and further S-isomer of an α,β-unsaturated isomer (4) is obtained without remarkable racemization, respectively.

### Example

The present invention is explained by the following examples, but the present invention should not be limited to these examples.

### Example 1

### (S)-3-(2,2-Dimethyl-1,3-dioxolan-4-yl)-2-propenoic acid methyl ester

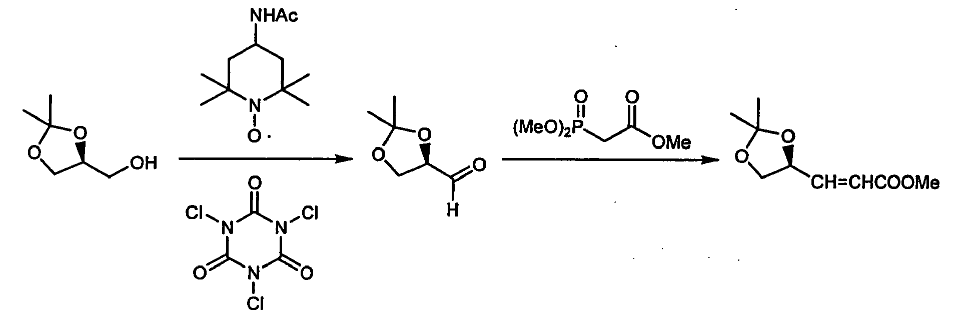

(S)-Glycerol acetonide (5.0g, 37.8mmol), sodium acetate (3.7g, 45.4mmol), 4-acetoamino-2,2,6,6-tetramethyl-1-piperidine-1-oxyl radical compound (TEMPO) (81mg, 0.38mmol) and ethyl acetate (50ml) were put in a 200ml-three neck flask. The suspension was cooled to less than 10°C in an ice bath and thereto was added trichloroisocyanuric acid (3.5g, 15.1mmol) divided by five portions. The insoluble salts are removed by filtration and to the filtrate were added 6M aqueous potassium carbonate solution (19ml) and phosphonoacetic acid trimethyl ester (8.3g, 45.4mmol). The mixture was stirred for 12 hours at room temperature. Thereto was added water (30ml). The mixture was separated by a separating funnel and the organic layer was concentrated to give a crude product. The crude product was purified by distillation to give the object ester compound (trans form/cis form=98/2) (5.8g, yield 82%).
¹H-NMR (CDCl₃/TMS) δ = 1.38 (s, 3H), 1.42 (s, 3H), 3.65 (dd, J=7.1, 8.2Hz, 1H), 3.72 (s, 3H), 4.16 (dd, J=6.6, 8.2Hz, 1H), 4.64 (m, 1H), 6.18 (dd, J=1.4, 15.6Hz, 1H), 6.87 (dd, J=5.6, 15.6Hz, 1H)
Optical rotary power [α]_{D}²²: + 46.0° (c=3.9, CHCl₃)

### Comparative Example 1.

### (S)-3-(2,2-Dimethyl-1,3-dioxolan-4-yl)-2-propenoic acid ethyl ester

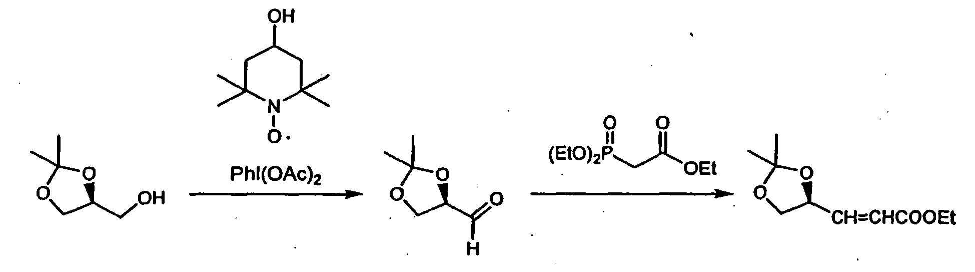

(S)-Glycerol acetonide (5.0g, 37.8mmol), 4-hydroxy TEMPO (130mg, 0.76mmol) and dichloromethane (50ml) were put in a 200-ml three neck flask. To the suspension was added diacetoxyiodobenzene (13.4g, 41.6mmol) and the mixture was stirred for about 3 hours while confirming disappearance of the starting material. Further sodium carbonate (12.0g, 113mmol) and phosphonoacetic acid triethyl ester (9.1g, 45.4mmol) were added thereto. The mixture was stirred for 16 hours at room temperature and thereto was added water (100ml). The mixture was separated by a separating funnel and the organic layer was concentrated to give a crude product. The crude product was purified by distillation to give the object ester compound (trans form/cis form=98/2) (6.6g, yield 87%).
¹H-NMR (CDCl₃/TMS) δ = 1.27 (t, J=7.1, 3H), 1.39 (s, 3H), 1.43 (s, 3H), 3.66 (dd, J=7.1, 8.1Hz, 1H), 4.16 (dd, J=6.6, 8.3Hz, 1H), 4.18 (q, J=7.1, 2H), 4.66 (m, 1H), 6.08 (dd, J=1.4, 15.6Hz, 1H), 6.86 (dd, J=5.7, 15.6Hz, 1H)
Optical rotary power [α]_{D}²⁰: + 44.0° (c=0.5, CHCl₃)

### Comparative Example 2

### (R)-3-(2,2-Dimethyl-1,3-dioxolan-4-yl)-2-propenoic acid methy ester

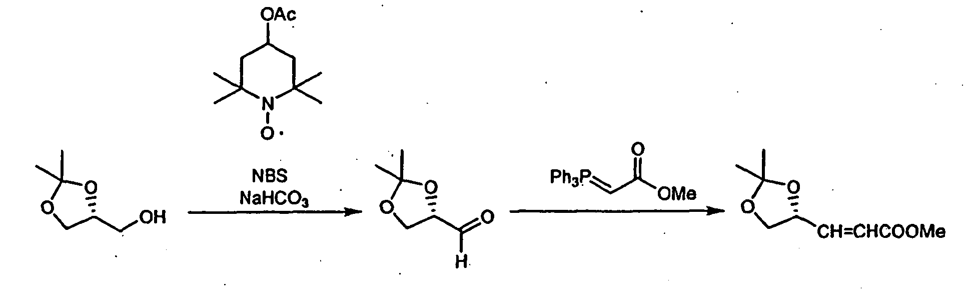

(R)-Glycerol acetonide (5.0g, 37.8mmol), sodium hydrogencarbonate (4.8g, 56.7mmol), 4-acetoxy TEMPO (41mg, 0.19mmol) and THF (50ml) were put in a 200-ml three neck flask. The suspension was cooled to less than 10°C in an ice bath and thereto was added NBS (8.1g, 45.4mmol) divided by three portions. The insoluble salts are removed by filtration and the filtrate was cooled to 0°C. Thereto was added methyl (triphenylphosphoranylidene)acetate (15.1g, 45.4mmol) and the mixture was stirred at 0°C for 16 hours. To the reaction mixture was added hexane (100ml) and the insoluble materials were filtered off and the filtrate was concentrated. The crude product was purified by silica gel chromatography to give the object ester (trans form/cis form =21/79) (5.3g, yield 75%).
¹H-NMR (CDCl₃/TMS) δ = 1.37 (s, 3H), 1.43 (s, 3H), 3.60 (dd, J=6.7, 8.2Hz, 1H), 3.70 (s, 3H), 4.36 (dd, J=6.9, 8.2Hz, 1H), 5.47 (apparent dq, J=1.7, 6.8Hz, 1H), 5.83 (dd, J=1.7, 6.8Hz, 1H), 6.35 (dd, J=6.6, 11.6Hz, 1H)
Optical rotary power [α]_{D}²⁰: -118.5° (c=1.8, CHCl₃)

### Example 2

### (S)-3-(2,2-Dimethyl-1,3-dioxolan-4-yl)-2-propenoic acid methyl ester

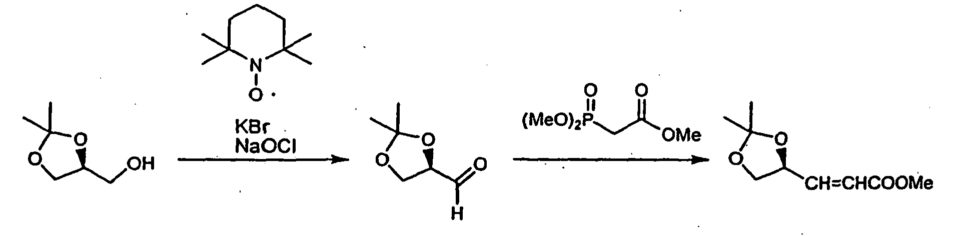

(S)-Glycerol acetonide (66.1g, 500mmol), TEMPO (0.78g, 5mmol), dichloromethane (170ml) and potassium bromide (5.95g, 50mmol) were dissolved in water (25ml) and the solution was put in a 1-L three neck flask. The solution was cooled to 0°C in an ice bath and therein was dissolved sodium hydrogencarbonate (9.35g, 111mmol). Thereto was dropped 1M aqueous sodium hypochlorite solution (550ml) which were adjusted to around pH 9.5 while the solution was kept at 10 to 15°C. Further sodium carbonate (207g, 1.5mol) and phosphonoacetic acid trimethyl ester (111g, 600mmol) were added thereto and the mixture was stirred for 12 hours at room temperature. The mixture was separated by a separating funnel and the organic layer was concentrated to give a crude product. The crude product was purified by distillation to give the object ester compound (trans form/cis form=98/2) (74.5g, yield 80%).
¹H-NMR (CDCl₃/TMS) δ = 1.38 (s, 3H), 1.42 (s, 3H), 3.65 (dd, J=7.1, 8.2Hz, 1H), 3.72 (s, 3H), 4.16 (dd, J=6.6, 8.2Hz, 1H), 4.64 (m, 1H), 6.18 (dd, J=1.4, 15.6Hz, 1H), 6.87 (dd, J=5.6, 15.6Hz, 1H)
Optical rotary power [α]_{D}²³: + 44.6° (c=3.0, CHCl₃)

The present invention is to provide a process for preparing an α,β-unsaturated ester (4) by oxidizing a glycerol derivative to prepare an aldehyde and then preparing the ester (4). Thus obtained compound (4) can be utilized as an intermediate for medicine in the organic synthesis industry.

It should not need to be stated that where the upper and lower limits of ranges herein are governed by different technical criteria, as they generally are, the upper and lower limits can be treated independently of one another.

## Claims

1. A process for preparing an α,β-unsaturated ester of the following compound (4), 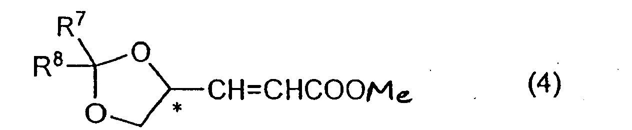 wherein R⁷ and R⁸ are methyl groups, which comprises oxidizing a glycerol derivative of the following formula (2), 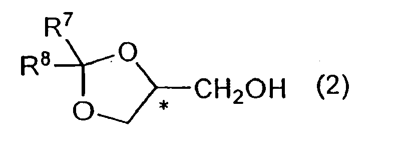 wherein R⁷ and R⁸ are the same as defined above, in the presence of a nitroxyl radical compound represented by the following (1), 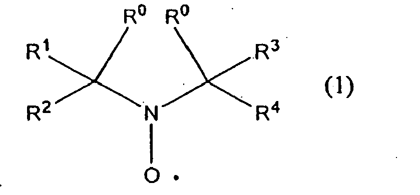 wherein R⁰ to R⁴ are the same or different and are straight or branched C₁₋₁₀ alkyl group, or two R⁰s may be combined together with the nitrogen atom in the molecule to form a 5 to 7 membered hetero ring,
and a co-oxidant, to prepare a glyceraldehyde of the following formula (3), 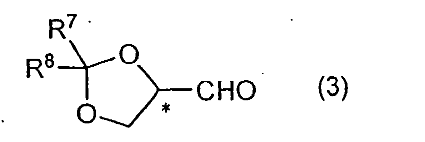 wherein R⁷ and R⁸ are the same as defined above, and then reacting the compound (3) with trimethyl phosphonoacetate to give the compound (4) without isolation of the compound (3).

2. A process of claim 1 wherein the nitroxyl radical compound is a compound of the following formula (1a), 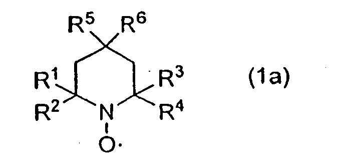 wherein R¹ to R⁴ are the same as defined above, R⁵ and R⁶ are both hydrogen atom or both C₁₋₆ alkoxy group, or one of R⁵ and R⁶ is hydrogen atom and the other is hydroxy group, C₁₋₆ alkoxy group, acyloxy group or acylamino group.

3. A process of claim 2 wherein the nitroxyl radical compound is a compound of the formula (1a) wherein R¹ to R⁴ are all methyl group, and R⁵ and R⁶ are both hydrogen atom, or one of R⁵ and R⁶ is hydrogen atom and the other is hydroxy group, methoxy group, acetoxy group, benzoyloxy group or acetamide group.

4. A process of any one of claims 1 to 3 wherein the co-oxidant is one or more selected from sodium hypochlorite, t-butyl hypochlorite, chlorine, bromine, iodine, bleaching powder, diacetoxyiodobenzene, m-chloroperbenzoic acid, N-chlorosuccinimide (NCS), dichlorodimethylhydantion, trichloroisocyanuric acid, N-bromoacetamide and N-bromosuccinimide (NBS).

5. A process of any one of claims 1 to 3 wherein the co-oxidant is sodium hypochlorite, trichloroisocyanuric acid, diacetoxyiodobenzene, N-bromoacetamide, N-bromosuccinimide (NBS) or a combination of N-chlorosuccinimide and a compound having bromide ion.

6. A process of any one of claims 1 to 3 wherein the co-oxidant is N-bromoacetamide, N-bromosuccimide (NBS) or a combination of N-chlorosuccinimide and a compound having bromide ion.

## Patentansprüche

1. Verfahren zur Herstellung eines a,β-ungesättigten Esters der nachstehenden Verbindung (4) 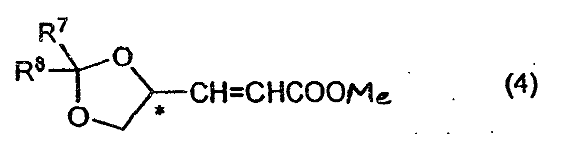 worin R⁷ und R⁸ Methylgruppen sind,
welches die Oxidation eines Glycerinderivats der nachstehenden Formel (2) 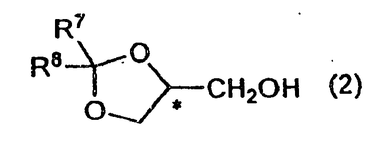 worin R⁷ und R⁸ gleich wie oben definiert sind,
in Gegenwart einer Nitroxylrestverbindung der nachstehenden Formel (1) 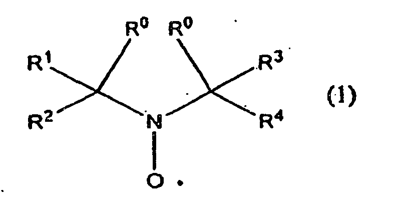 worin R⁰ bis R⁴ gleich oder unterschiedlich sind und eine unverzweigte oder verzweigte C₁₋₁₀-Alkylgruppe sind oder zwei R⁰ mit dem Stickstoffatom im Molekül kombiniert sein können, um einen 5- bis 7-gliedrigen Heteroring zu bilden,
und eines Co-Oxidans zur Herstellung eines Glyceraldehyds der nachstehenden Formel (3), 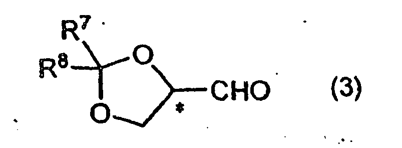 worin R⁷ und R⁸ gleich wie oben definiert sind,
und anschließend die Umsetzung der Verbindung (3) mit Trimethylphosphonoacetat, um die Verbindung (4) zu ergeben, ohne Isolierung der Verbindung (3) umfasst.

2. Verfahren nach Anspruch 1, worin die Nitroxylrestverbindung eine Verbindung der nachstehenden Formel (1a) ist, 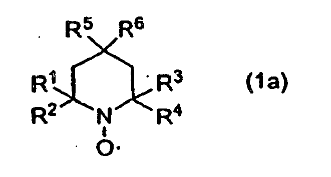 worin R¹ bis R⁴ gleich wie oben definiert sind, R⁵ und R⁶ beide ein Wasserstoffatom oder eine C₁₋₆-Alkoxygruppe sind oder einer aus R⁵ und R⁶ ein Wasserstoffatom und der andere eine Hydroxygruppe, C₁₋₆-Alkoxygruppe, Acyloxygruppe oder Acylaminogruppe ist.

3. Verfahren nach Anspruch 2, worin die Nitroxylrestverbindung eine Verbindung der Formel (1a) ist, worin R¹ bis R⁴ alle eine Methylgruppe sind und R⁵ und R⁶ beide ein Wasserstoffatom sind oder einer aus R⁵ und R⁶ ein Wasserstoffatom und der andere eine Hydroxygruppe, Methoxygruppe, Acetoxygruppe, Benzoyloxygruppe oder Acetamidgruppe ist.

4. Verfahren nach einem der Ansprüche 1 bis 3, worin das Co-Oxidans eines oder mehrere ist, das/die aus Natriumhypochlorit, t-Butylhypochlorit, Chlor, Brom, Iod, Bleichpulver, Diacetoxyiodbenzol, m-Chlorperbenzoesäure, N-Chlorsuccinimid (NCS), Dichlordimethylhydantoin, Trichlorisocyanursäure, N-Bromacetamid oder N-Bromsuccinimid (NBS) ausgewählt ist/sind.

5. Verfahren nach einem der Ansprüche 1 bis 3, worin das Co-Oxidans Natriumhypochlorit, Trichlorisocyanursäure, Diacetoxyiodbenzol, N-Bromacetamid, N-Bromsuccinimid (NBS) oder eine Kombination aus N-Chlorsuccinimid und einer Verbindung mit einem Bromidion ist.

6. Verfahren nach einem der Ansprüche 1 bis 3, worin das Co-Oxidans N-Bromacetamid, N-Bromsuccinimid (NBS) oder eine Kombination aus N-Chlorsuccinimid und einer Verbindung mit einem Bromidion ist.

## Revendications

1. Procédé de préparation d'un ester α,β-insaturé du composé suivant (4); 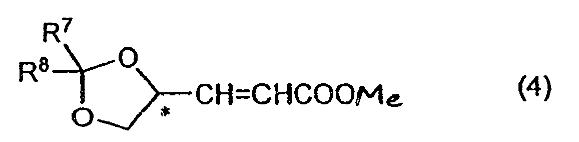 où R⁷ et R⁸ sont des groupes méthyles, qui comprend l'oxydation d'un dérivé de glycérol de la formule suivante (2), 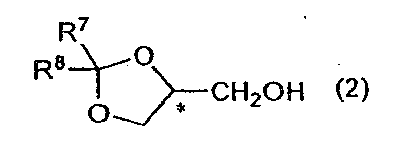 où R⁷ et R⁸ sont les mêmes que définis ci-dessus, en présence d'un composé de radical nitroxyle représenté par la formule suivante (1), 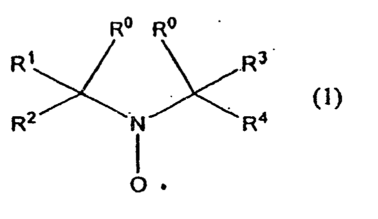 où R⁰ à R⁴ sont les mêmes ou différents et sont des groupes alkyles C₁₋₁₀ droites ou branchées, ou bien deux R⁰s peuvent être combinés ensemble avec l'atome d'azote dans la molécule pour former un hétérocycle de 5 à 7 membres,
et un co-oxydant, pour préparer un glycéraldéhyde de la formule suivante (3), 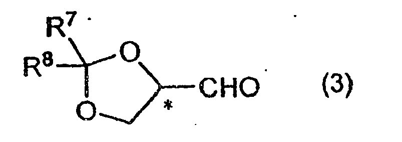 où R⁷ et R⁸ sont les mêmes que définis ci-dessus, et à faire réagir ensuite le composé (3) avec du triméthyl phosphonoacétate pour donner le composé (4) sans isolation du composé (3).

2. Procédé selon la revendication 1, dans lequel le composé de radical nitroxyle est un composé de la formule suivante (1a), 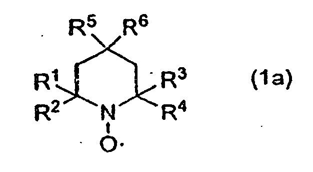 où R¹ à R⁴ sont les mêmes que définis ci-dessus, R⁵ et R⁶ sont tous les deux un atome d'hydrogène ou tous les deux un groupe alcoxy C₁₋₆, ou bien un parmi R⁵ et R⁶ est un atome d'hydrogène et l'autre est un groupe hydroxy, groupe alcoxy C₁₋₆, groupe acyloxy ou groupe acylamino.

3. Procédé selon la revendication 2, dans lequel le composé de radical nitroxyle est un composé de la formule (1a), où R¹ à R⁴ sont tous un groupe méthyle, et R⁵ et R⁶ sont tous les deux un atome d'hydrogène, ou bien un parmi R⁵ et R⁶ est un atome d'hydrogène et l'autre est un groupe hydroxy, groupe méthoxy, groupe acétoxy, groupe benzoyloxy ou groupe acétamide.

4. Procédé selon l'une quelconque des revendications 1 à 3, dans lequel le co-oxydant est un ou plusieurs sélectionnés parmi le sodium hypochlorite, t-butyl hypochlorite, chlore, brome, iode, poudre de blanchiment, diacétoxyiodobenzène, acide m-chloroperbenzoique, N-chlorosuccinimide (NCS), dichlorodiméthylhydantoïne, acide trichloroisocyanurique, N-bromoacétamide et N-bromosuccinimide (NBS).

5. Procédé selon l'une quelconque des revendications 1 à 3, dans lequel le cooxydant est du sodium hypochlorite, acide trichloroisocyanurique, diacétoxyiodobenzène, N-bromoacétamide, N-bromosuccinimide (NBS) ou une combinaison de N-chlorosuccinimide et d'un composé ayant un ion bromure.

6. Procédé selon l'une quelconque des revendications 1 à 3, dans lequel le co-oxydant est un N-bromoacétamide, N-bromosuccinimide (NBS) ou bien une combinaison de N-chlorosuccinimide et d'un composé ayant un ion bromure.
